# EUROPEAN PATENT SPECIFICATION

(11) **EP 2 551 342 B1**
(45) Date of publication and mention of the grant of the patent: **02.05.2018**
(21) Application number: 11759703.9
(22) Date of filing: 22.03.2011
(51) Int. Cl.: C12N 5/077, C12N 5/02, C07K 14/495, A61K 35/32, A61P 19/00, A61F 2/30, C12N 5/071, A61K 35/12, C12N 5/0775

(54) **METHOD FOR DIFFERENTIATING CARTILAGE CELLS, BONE CELLS, NERVE CELLS OR FAT CELLS FROM HUMAN INFERIOR TURBINATE MESENCHYMAL STROMAL CELLS**
VERFAHREN ZUR DIFFERENZIERUNG VON KNORPELZELLEN, KNOCHENZELLEN, NERVENZELLEN ODER FETTZELLEN AUS MENSCHLICHEN MESENZYMALEN STAMMZELLEN AUS DER UNTEREN NASENMUSCHEL
MÉTHODE DE DIFFÉRENCIATION DE CELLULES CARTILAGINEUSES, DE CELLULES OSSEUSES, DE CELLULES NERVEUSES OU D'ADIPOCYTES À PARTIR DE CELLULES STROMALES MÉSENCHYMATEUSES HUMAINES DU CORNET INFÉRIEUR

(30) Priority: 22.03.2010 KR 20100025207; 04.03.2011 KR 20110019208
(43) Date of publication of application: 30.01.2013
(73) Proprietor: Catholic University Industry Academic Cooperation Foundation, Seoul 137-701 (KR)
(72) Inventor: KIM, Sung Won, Seoul 135-789 (KR)
(74) Representative: Appleyard Lees IP LLP
(86) International application number: PCT/KR2011/001947
(87) International publication number: WO 2011/118954

(56) References cited:
- KIM S W ET AL: "Induction of chondrogenic differentiation in cultured fibroblasts isolated from the inferior turbinate", OTOLARYNGOLOGY AND HEAD AND NECK SURGERY, ROCHESTER, US, vol. 139, no. 1, 1 July 2008 (2008-07-01), pages 143-148, XP022849544, ISSN: 0194-5998, DOI: 10.1016/J.OTOHNS.2008.04.015 [retrieved on 2008-06-26]
- ALLISON A. WORSTER ET AL: "Chondrocytic differentiation of mesenchymal stem cells sequentially exposed to transforming growth factor-[beta]1 in monolayer and insulin-like growth factor-I in a three-dimensional matrix", JOURNAL OF ORTHOPAEDIC RESEARCH, vol. 19, no. 4, 1 July 2001 (2001-07-01), pages 738-749, XP55071743, ISSN: 0736-0266, DOI: 10.1016/S0736-0266(00)00054-1
- LEE JIN WOO ET AL: "Chondrogenic differentiation of mesenchymal stem cells and its clinical applications", YONSEI MEDICAL JOURNAL, YONSEI UNIVERSITY, KI, vol. 45, no. Suppl. S, 30 June 2004 (2004-06-30), pages 41-47, XP002511546, ISSN: 0513-5796
- KIM ET AL.: 'Induction of chondrogenic differentiation in cultured fibroblasts isolated from the inferior turbinate' OTOLARYNGOLOGY-HEAD AND NECK SURGERY vol. 139, July 2008, pages 143 - 148
- SUNG WON KIM: 'Current trends in tissue regeneration using fibroblasts' KOREAN JOURNAL OF OTORHINOLARYNGOLOGY-HEAD AND NECK SURGERY vol. 5, no. 1, 2008, pages 114 - 119
- COLLEONI ET AL.: 'Establishment, differentiation, electroporation, viral transduction, and nuclear transfer of bovine and porcine mesenchymal stem cells' CLONING AND STEM CELLS vol. 7, no. 3, 2005, pages 154 - 166
- KIM ET AL.: 'Inferior turbinate: new source of mesechymal stromal cells' OTOLARYNGOLOGY-HEAD AND NECK SURGERY vol. 143, 10 August 2010, page P151
- KUNDROTAS G: "Surface markers distinguishing mesenchymal stem cells from fibroblasts", ACTA MEDICA LITUANICA, vol. 19, 2012, pages 75-79,
- ECKHARD ALT ET AL: "Fibroblasts share mesenchymal phenotypes with stem cells, but lack their differentiation and colony-forming potential", BIOLOGY OF THE CELL, vol. 103, no. 4, 1 April 2011 (2011-04-01) , pages 197-208, XP055264640, FR ISSN: 0248-4900, DOI: 10.1042/BC20100117
- HALFON SVETLANA ET AL: "Markers distinguishing mesenchymal stem cells from fibroblasts are downregulated with passaging.", STEM CELLS AND DEVELOPMENT JAN 2011, vol. 20, no. 1, January 2011 (2011-01), pages 53-66, ISSN: 1557-8534

<110> Industry Academic Cooperation Foundation of Catholic University
<120> Method of inducing chondrogenic, osteogenic, neurogenic or adipocytic differentiation of human turbinate mesenchymal stromal cells
<150> KR10-2010-0025207
   <151> 2010-03-22
<150> KR10-2011-0019208
   <151> 2011-03-04
<160> 18
<170> KopatentIn 1.71
<210> 1
   <211> 20
   <212> DNA
   <213> Artificial Sequence
<220>
   <223> beta actin F primer
<400> 1
   gcccctccat cgtccaccgc 20
<210> 2
   <211> 20
   <212> DNA
   <213> Artificial Sequence
<220>
   <223> beta actin R primer
<400> 2
   gggcacgaag gctcatcatt 20
<210> 3
   <211> 20
   <212> DNA
   <213> Artificial Sequence
<220>
   <223> type II collagen F primer
<400> 3
   tttcccaggt caagatggtc 20
<210> 4
   <211> 20
   <212> DNA
   <213> Artificial Sequence
<220>
   <223> type II collagen R primer
<400> 4
   cttcagcacc tgtctcacca 20
<210> 5
   <211> 21
   <212> DNA
   <213> Artificial Sequence
<220>
   <223> aggrecan F primer
<400> 5
   gcagagacgc atctagaaat t 21
<210> 6
   <211> 22
   <212> DNA
   <213> Artificial Sequence
<220>
   <223> aggrecan R primer
<400> 6
   ggtaattgca gggaacatca tt 22
<210> 7
   <211> 18
   <212> DNA
   <213> Artificial Sequence
<220>
   <223> COL1 F primer
<400> 7
   tgacgagacc aagaactg 18
<210> 8
   <211> 20
   <212> DNA
   <213> Artificial Sequence
<220>
   <223> COL1 R primer
<400> 8
   ccatccaaac cactgaaacc 20
<210> 9
   <211> 21
   <212> DNA
   <213> Artificial Sequence
<220>
   <223> OC F primer
<400> 9
   atgagagccc tcacactcct c 21
<210> 10
   <211> 21
   <212> DNA
   <213> Artificial Sequence
<220>
   <223> OC R primer
<400> 10
   cgggccgtag aagcgccgat a 21
<210> 11
   <211> 21
   <212> DNA
   <213> Artificial Sequence
<220>
   <223> BSP F primer
<400> 11
   gctcagcatt ttgggaatgg c 21
<210> 12
   <211> 21
   <212> DNA
   <213> Artificial Sequence
<220>
   <223> BSP R primer
<400> 12
   ctgcattggc tccagtgaca c 21
<210> 13
   <211> 21
   <212> DNA
   <213> Artificial Sequence
<220>
   <223> Runx2 F primer
<400> 13
   gtggacgagg caagagtttc a 21
<210> 14
   <211> 21
   <212> DNA
   <213> Artificial Sequence
<220>
   <223> Runx2 R primer
<400> 14
   tggcaggtag gtgtggtagt g 21
<210> 15
   <211> 25
   <212> DNA
   <213> Artificial Sequence
<220>
   <223> Osx F primer
<400> 15
   cttcagtctt cccaacttct tacac 25
<210> 16
   <211> 25
   <212> DNA
   <213> Artificial Sequence
<220>
   <223> Osx R primer
<400> 16
   acaaattggg ttagctacat ctctg 25
<210> 17
   <211> 20
   <212> DNA
   <213> Artificial Sequence
<220>
   <223> BMP-2 F primer
<400> 17
   ttgcgcctgc tcagcatgtt 20
<210> 18
   <211> 22
   <212> DNA
   <213> Artificial Sequence
<220>
   <223> BMP-2 R primer
<400> 18
   catcttgcat ctgttctcgg aa 22

## Description

### [Technical Field]

The present invention relates to a method for differentiating human turbinate mesenchymal stromal cells into cartilage cells, bone cells, nerve cells, or fat cells and to a pharmaceutically composition including the cartilage cells, the bone cells, the nerve cells, or the fat cells that are differentiated from the human turbinate mesenchymal stromal cells as active ingredients.

### [Background Art]

Therapies that replace tissues or organs having damaged functions due to diseases or injuries with similar autologous tissues or organs having similar functions have developed according to advances in medicine. Unfortunately, the therapies cause problems, such as defects of donor sites. Recently, in order to address the problem, a tissue engineering technique including a step of obtaining a little cell from the donor sites, a step of proliferating the cell using a cell culture technique, and then a step of transplanting the proliferated cell has been attempted to replace an organ transplantation or a tissue transplantation.

Since cartilage that is a support structure of human body has a very low ability to reconstruct or regenerate, a therapy method through a culture of cartilage cells for reconstructing a naturally damaged or acquired damaged cartilage tissues are being developed. Especially, cartilage is an avascular tissue of homogeneous cartilage cells, so that its cell isolation and culture in vitro are relatively easy and it has an advantageous condition for a research of cell differentiation. Therefore, it has been a model for a research about regenerated tissues. Cartilage having a small size can be easily obtained from various parts of the body. Especially, septal cartilage can be obtained without serious complications of the donor sites as hyaline cartilage, and has most similar strength or histological density to an original cartiliage tissue when culturing and proliferating using the tissue engineering technique. Unfortunately, the adult cells are also limited to give, so that a research using stem cells for replacing it has been attempled.

In order for a deficit or a reconstruction of bone tissues forming the skeletal structure of the human body, an autograft transplantation or an allograft transplantation is being used for equal to or greater than 90% and a natural material or a synthetic biomaterial is being used only for approximately 10%. The transplanted graft may be suitable for reconstructing defected bone sites, but its use is limited. In the case of the autograft transplantation, there are side effects due to a limit of donor sites and a deficit of donor sites, and in the case of the allograft transplantation, a bone graft can be more easily obtained, but the graft without a biological activity may be used, and the possibility of introducing diseases from a donor and an immune rejection response may be experienced. In addition, the biomaterial that is often used along with the allograft transplantation can be used in a large quantity and play a role as scaffold to be introduced by a growth of the tissue, but the role for an regeneration of human skeletal structure is limited because there are no active cells.

Recently, in order to address the problems, a tissue engineering technique including a step of obtaining a little cell from the donor sites, a step of proliferating the cell using a cell culture technique, and then a step of transplanting the proliferated cell has been attempted to replace organ transplantation or tissue transplantation. For a method for using osteoblast as a cell source for reconstructing a defected bone tissue, the osteoblast is not appropriate as a cell source for an osteoanagenesis, because the osteoblast is difficult to proliferate in vitro. For such a reason, the need for an alternative cell source that can have similar in function to osteoblast is becoming the main issue, so that the research about bone tissue engineering using mesenchymal stromal cells having characteristics of stem cells as a cell source has been attempted.

Mesenchymal stromal cells having characteristics of stem cells can be obtained from bone marrow, resorbed fat tissues, and the like, which are an adult tissue and can be differentiated into various kinds of cells, so that many studies about the mesenchymal stromal cells are being carried out in the field of tissue engineering and regenerative medicine. Bone marrow-derived mesenchymal stromal cells are the best material for differentiating into various cells, but it is difficult to obtain and only part of the bone marrow components is mesenchymal stromal cells having the characteristics of stem cells. Accordingly, more research is needed to use it clinically.

Meanwhile, recently, research about fat tissues as donor sites of mesenchymal stromal cells is being carried out, actively. And also, a technique for making a cartilage specific matrix, such as type II and type VI collagens, and chondroitin 4-sulfate has been developed by performing a three-dimensional culture inducing cartilage cell differentiation with alginate as scaffold in the experiment using mesenchymal stromal cells isolated from a human subcutaneous fatty tissue. Unfortunately, the technique has low degree of cartilage-specific matrix production and cannot easily obtain the cartilage-specific matrix as compared with the bone marrow-derived mesenchymal stromal cells. Accordingly, the researches on source of mesenchymal stromal cells having characteristics of stem cells and multipotency using the same are needed.

Worster, A. A., et. al., (2001) Journal of Orthopaedic Research, Vol 19, Issue 4, July 2001, 738-749 discloses a study evaluating chondrogenesis of mesenchymal progenitor stem cells (MSCs) cultured initially under pre-confluent monolayer conditions exposed to transforming growth factor-β1 (TGF-β1), and subsequently in three-dimensional cultures containing insulin-like growth factor I (IGF-I) and indicated that although MSC differentiation was less than complete when compared to mature chondrocytes, chondrogenesis was observed in IGF-I supplemented cultures, particularly when used in conjunction with TGF-β1 pretreatment. Kim S W et al, OTOLARYNGOLOGY AND HEAD AND NECK SURGERY, 2008, vol. 139: pages 143-148, discloses the induction of chondrogenic differentiation in cultured fibroblasts isolated from the inferior turbinate.

### [Disclosure]

### [Technical Problem]

While the inventors were looking for a source of new mesenchymal stromal cells having mutilpotency, the inventors used human turbinate mesenchymal stromal cells in view of the recycling of discarded tissues and can establish the conditions that can differentiate human inferior turbinate mesenchymal stromal cells into cartilage cells, bone cells, fat cells, or nerve cells to use human inferior turbinate mesenchymal stromal cells for medical purposes, and then completed the present invention.

Accordingly, it is an object to provide a method for differentiating human inferior turbinate mesenchymal stromal cells into cartilage cells, bone cells, or nerve cells, the method including a step of culturing the human inferior turbinate mesenchymal stromal cells in a medium including nerve growth factors.

In addition, another object is to provide a composition for preventing or treating bone, cartilage, or nerve injuries or diseases, the composition including cartilage cells, bone cells, or nerve cells that are differentiated from the human turbinate mesenchymal stromal cells as an active ingredient.

In addition, still another object is to provide a method for isolating mesenchymal stromal cells from isolated human inferior turbinate tissues.

In addition, still another object is to provide a method for differentiating human turbinate mesenchymal stromal cells into fat cells, the method including a step of culturing the human turbinate mesenchymal stromal cells in a medium for the fat cells differentiation.

In addition, still another object is to provide a composition for transplanting fat cells for regenerating skin tissues, the composition including the fat tissues that are differentiated from the human turbinate mesenchymal stromal cells.

In addition, still another object is to a method for treating cartilage injuries or diseases, the method including a step of treating the cartilage cells that are differentiated according to the method of the present invention to an object requiring the same.

In addition, still another object is to a method for treating bone injuries or diseases, or metabolic bone diseases, the method including a step of treating the bone cells that are differentiated according to the method of the present invention to an object requiring the same.

In addition, still another object of the present invention is to a method for treating nerve injuries or diseases, the method including a step of treating the nerve cells that are differentiated according to the method of the present invention to an object requiring the same.

In addition, still another object of the present invention is to a method for transplanting fat cells for regenerating skin tissue, the method including a step of treating the fat cells that are differentiated according to the method of the present invention to an object requiring the same.

### [Technical Solution]

Aspects of the present invention are described with reference to the appended claims.

Disclosed is a method for differentiating human turbinate mesenchymal stromal cells (hTMSCs) into cartilage cells, the method including a step of culturing the human mesenchymal stromal cells in a cartilage cell differentiation medium including transforming growth factor-β1 (TGF-β1) or insulin like growth factor-I (IGF-I).

The medium may further include at least one selected from the group consisting of dexamethasone, ascorbate, insulin-transferrin sodium selenite, sodium pyrubate, proline, and L-glutamine.

The culture may be a co-culture with septal cartilage cells that are isolated from human septal cartilage and then cultured.

Disclosed is a composition for preventing or treating cartilage injuries or diseases, the composition including the cartilage cells that are differentiated by using the method for differentiating into the cartilage cells as described, and also a method for treating cartilage injuries or diseases, the method including a step of treating the cartilage cells thus differentiated to an object requiring the same.

The cartilage injuries or diseases may be degenerative arthritis or rheumatoid arthritis.

Disclosed is a method for manufacturing an artificial joint, the method including a step of using the cartilage cells differentiated by using the method for differentiating cartilage cells described, and an artificial joint being manufactured from the above method.

Additionally disclosed is a method for differentiating human turbinate mesenchymal stromal cells (hTMSCs) into bone cells, the method including a step of culturing the human turbinate mesenchymal stromal cells in a medium without a fetal bovine serum (FBS).

The medium may further include at least one selected from the group consisting of penicillin, streptomycin, dexamethasone, β-glycerophosphate, and ascorbate-2-phosphate.

Disclosed is a composition for preventing or treating bone injuries or diseases, or metabolic bone diseases, the composition including the bone cells that are differentiated by using the method for differentiating into the bone cells as described, and also a method for treating bone injuries or diseases, or metabolic bone diseases, the method including a step of treating the bone cells thus differentiated to an object requiring the same.

Also disclosed is a method for differentiating human turbinate mesenchymal stromal cells (hTMSCs) into nerve cells, the method including a step of culturing the human turbinate mesenchymal stromal cells in a neural differentiation medium including a glial-derived neurotrophin factor (GDNF), a brain-derived neurotrophin factor (BDNF), a neurotrophin-3 (NT3), a B-27 supplement, and L-glutamine, a composition for preventing or treating nerve injury or disease, the composition including the nerve cells differentiated from the method for differentiating the nerve cells, and also a method for treating nerve injuries or diseases, the method including a step of administrating the nerve cells thus differentiated to an object requiring the same.

Disclosed is a method for differentiating human terbinate mesenchymal stromal cells, the method including a step of culturing in a fat cell differentiation medium including insulin, indomethacin, dexamethasone, 3-isobutyl-1-methylxanthine, and a fetal bovine serum (FBS).

Additionally disclosed is a composition for transplanting fat cells for regenerating skin tissues, the composition including the fat cells differentiated by using the method described, and a method for transplanting fat cells for regenerating skin tissues, the method including a step of treating the fat cells thus differentiated to an object requiring the same.

Disclosed is a method for isolating mesenchymal stromal cells from isolated human inferior turbinate tissues, the method including a step of cutting the isolated human inferior turbinate tissues; a step of culturing the inferior turbinate tissues by adhering the inferior turbinate tissues to a culture dish; a step of removing un-adhered cells; and a step of obtaining human turbinate mesenchymal stromal cells adhered on the dish.

By confirming human turbinate mesenchymal stromal cells having ability capable of mutipotency into bone cells, cartilage cells, or nerve cells, it could be confirmed that the bone cells, cartilage cells, or nerve cells differentiated from the human inferior turbinate mesenchymal stromal cells as described can be used in the fields of cell replacement therapy and regenerative medicine for treating diseases caused by the damage of the cells, and the human inferior turbinate tissues as decribed can be easily obtained as the source of the cells from the discarded tissues during inferior turbinate surgery.

### [Description of Drawings]

FIG. 1 shows a photograph of human turbinate mesenchymal stromal cells after 2 weeks of a first explants culture;
FIG. 2 shows a result of histological examination of hTMSCs-inoculation PCL scaffold product during 2 weeks of culture, in which micronodule-like colonies of hTMSCs are scattered in a PCL scaffold pore, and A represents a H&E staining, B represents a Toluidine blue-O staining, and C represents an immunofluorescence staining to Type II collagen;
FIG. 3 shows a result of histological examination of hTMSCs and hSCs-inoculations PCL scaffold product during 2 weeks of culture, in which cartilage cells including mixed hTMSCs and extracellular matrices are scattered in a PCR scaffold pore, and A represents a H&E staining, B represents a Toluidine blue-O staining, and C represents an immunofluorescence staining to Type II collagen;
FIG. 4 shows a histological result of hSCs-inoculation PCL scaffold product during 2 weeks of culture, and A represents a H&E staining, B represents a Toluidine blue-O staining, and C represents an immunofluorescence staining to Type II collagen;
FIG. 5 shows RT-PCR analysis of Type II coallgen and aggrecan mRNA expression in hTMSCs and/or hSCs-inoculations PCL scaffold product at 2 weeks;
FIG. 6 shows Type II collagen mRNA level using a real time PCR after 2 weeks of inoculation;
FIG. 7 shows a result of confirming cartilage-specific Type II collagen from hTMSCs-inoculation PCL scaffold product through a western blot method during 2 weeks of culture;
FIG. 8 shows results of confirming mRNA expression levels of Type I collagen, a bone sialoprotein (BSP), Runt-related transcription factor 2 (Runx2), bone morphogenetic protein-2(BMP-2), osterix(Osx), and osteocalcin(OC), that are obtained by performing a RT-PCR analysis in bone cells-differentiated hTMSCs;
FIG. 9 shows bone cells differentiation of hTMSCs, and A represents an alkaline phosphatase staining at 1 week of culture; B represents an alizarin red staining at 1 week of culture; C represents an alkaline phosphatase staining at 2 weeks of culture; and D represents an alizarin red staining at 2 weeks of culture;
FIG. 10 shows nerve cells differentiation of hTMSCs at 14 days of culture;
FIG. 11 shows a fluorescence-activated cell sorting (FACS) analysis of hTMSCs; and
FIG. 12 shows a photograph of observing a differentiation from hTMSCs into fat cells through a oil red staining.

### [Best Mode]

Disclosed is a method for differentiating human turbinate mesenchymal stromal cells into bone cells, cartilage cells, or nerve cells, the method including a step of culturing the human turbinate mesenchymal stromal cells in a medium for differentiating into the bone cells, the cartilage cells, or the nerve cells.

Human inferior turbinate used for differentiating into each cell is tissue that exists in noses of human and is submucosa-rich tissue, in which a great quantity of reticular venous sinuses is distributed inside the human inferior turbinate, and many mesenchymal stromal cells exist around the human inferior turbinate. It can be used that the human inferior turbinate is obtained during a surgical removal of inferior turbinate that is performed in the field of otolaryngology so as to improve a symptom of nasal congestion.

In addition, the tissue, which is removed as biopsy during a short period time without any pain from an outpatient in the field of otolaryngology not an operating room, can be used.

Accordingly, the provision of a material is disclosed for autologous giving and autoplasty by using tissue that is removed and discarded during many surgery processes performed for improving human anatomy with the purpose of alleviating symptoms, and can contribute to a recycling of discarded tissue and an establishment of cell bank and customized tissue for patients by using the discarded tissue.

For this reason, the inventors isolated mesenchymal stromal cells from human inferior turbinate tissues and then investigated whether human turbinate mesenchymal stromal cells (hTMSCs) can be differentiated into cartilage cells, bone cells, and nerve cells.

First, in order to confirm whether mesenchymal stromal cells can be differentiated into cartilage for the mesenchymal stromal cells isolated from human inferior turbinate tissues, human turbinate mesenchymal stromal cells are cultured in a culture medium including growth factors, such as TGF-β1 and IGF-I, and as a result, the fact that the human turbinate mesenchymal stromal cells can generate a cartilage-specific matrix can be confirmed through a toluidine blue staining, an immunehistologic staining method to Type II collagen, and a RT-PCR method.

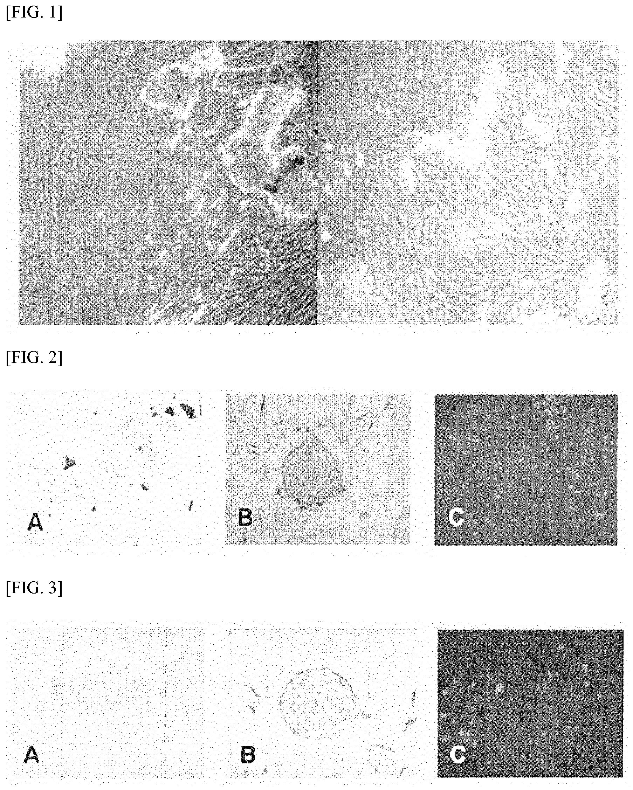

In addition, as a result of investigating a differentiation of cartilage by co-culturing mesenchymal stromal cells isolated from human inferior turbinate tissues with human septal cartilage cells that are known as a slow proliferation and a generation of many cartilage-specific extracellular matrix, it also generates a cartilage-specific matrix (see FIGS. 2 to 5).

Accordingly, a method is disclosed for differentiating human turbinate mesenchymal stromal cells (hTMSCs) into cartilage cells, the method including a step of culturing the human turbinate mesenchymal stromal cells in a cartilage differentiation medium including a transforming growth factor-β1 (TGF-β1) or an insulin like growth factor-I (IGF-I). The medium used for the culture may further include at least any one selected from the group consisting of dexamethasone, ascorbate, insulin-transferrin sodium selenite, sodium pyrubate, praline, and L-glutamine, in addition to a transforming growth factor-β1 (TGF-β1) or an insulin like growth factor-I (IGF-I).

In addition, the human turbinate mesenchymal stromal cells used can be obtained by isolating inferior turbinate tissues by using a method, such as a washing, a cutting, and the like, and then culturing the inferior turbinate tissues in a medium for culturing mesenchymal stromal cells. The isolated mesenchymal stromal cells may be used in 2 to 5 times of subcultures, preferably 3 to 4 times of subcultures, and more preferably 3 times of subcultures.

Accordingly, there is disclosed a method for isolating mesenchymal stromal cells from isolated human inferior turbinate tissues, the method including a step of cutting the isolated human inferior turbinate tissues; a step of culturing the inferior turbinate tissues by adhering the inferior turbinate tissues to a culture dish; a step of removing un-adhered cells; and a step of obtaining human turbinate mesenchymal stromal cells adhered on the dish.

In addition, for a method for differentiating the turbinate mesenchymal stromal cells into cartilage cells, first mesenchymal stromal cells are isolated from inferior turbinate, and then the isolated mesenchymal stromal cells may be differentiated into cartilage cells by treating cartilage growth factors to a culture medium, in which cells are proliferated. In addition, the differentiation into cartilage cells may be stimulated by co-culturing the turbinate mesenchymal stromal cells with septal cartilage cells that are isolated from human septal cartilage and then cultured.

For the turbinate mesenchymal stromal cells, all of the methods for isolating mesenchymal stromal cells that are known in the prior art may be used. It may be obtained by collecting a cell fraction by performing a density gradient centrifuge with the cells taken from vertebrate; isolating mesenchymal stromal cells only; and then culturing it. In this case, the density gradient centrifuge is a method for obtaining only the cell fraction having a constant specific gravity, and also process for rotating the cell at constant rate during enough time so as to place the cell at a position corresponding to its specific gravity in a solution for the density gradient centrifuge, such as ficol, percol, and hystopaque.

The turbinate mesenchymal stromal cells obtained from such the method may be differentiated into cartilage cells by treating cartilage growth factors to a culture medium, in which cells are proliferated after, and then culturing.

In this case, as such a culture medium, all the culture mediums are available as long as they are commonly for culturing an animal cell, but are not limited, a DMEM, an alpha-DMEM, an Eagle's basal medium, a RPMI 1640 medium, a Neutral progenitor cell basal medium (NPBM, SCIonetics), and the like can be used.

Cartilage has the greatest weight bearing of human anatomies, and is easily exposed to an injury due to abrasion. The articular cartilage is hyaline cartilage, and is composed of cartilage cells and a rich extracellular matrix. The cartilage cells are presented in less than 10% of total volume of cartilage, and synthesize and secrete the extracellular matrix, so that it plays an important role in the maintenance of the articular cartilage. In addition, unusually, the articular cartilage is non-renewable tissue itself after injuring because it does not have blood vessels, nerve tissues, and lymphoid tissue. As for a current medical method, a method for normally regenerating the articular cartilage is highly limited. A regeneration of cartilage tissue is recently attempted by autologus chondrocyte transplantation, so that the autologous cartilage cells transplantation is known as a very effective operation clinically. However, the transplantation cannot be used on a cartilage region of widespread damage and also is limited according to a damage site of the articular cartilage, the age of the patient, and the like.

Accordingly, a tissue engineering method for regenerating cartilage has been recently developed by efforts to address the problems described above and develop a safer operation (kang et al. Tissue Engineering, 11(3-4), pp.438-447, 2005), and a research relating to a manufacture of the artificial cartilage is actively being carried out now in foreign countries. Especially, a cartilage cells-differentiated stem cell and biodegradable scaffold are essential for manufacturing tissue engineered articular cartilage of cartilage cells differentiated from stem cells. In addition, the cartilage cell-differentiated stem cell induces a differentiation into cartilage cells while single nucleus cells are cultured after isolating from bone marrow and ideal cartilage cells should have good ability of proliferation and maintain Type II collagen that is a specific phenotype of cartilage cell.

Meanwhile, human turbinate mesenchymal stromal cells that can be differentiated into cartilage cells, have excellent proliferation ability, and maintain cartilage cell-specific Type II collagen, so that it can be used as an active ingredient of cell composition for cell replacement therapy for treating cartilage injuries, and the like.

Provided is a composition for treating cartilage injuries or diseases, the composition including the cartilage cells that are differentiated according to the method as an active ingredient. The cartilage injuries or diseases may include, but is not limited, degenerative arthritis, rheumatoid arthritis, and an external injury.

The composition including the cartilage cells that are differentiated an active ingredient may be injected directly in joint of the patient according to a known method, or transplanted to a region requiring it along with scaffold after a three-dimensional culture (Kim, G. et al, J. Vet. Med. Sci., 66:263, 2004, Lee,J.W. et al., Yonsei Med. J., 30:41, 2004). The number of the administrated cell is preferably controlled in accordance with various relevant factors, such as a disease to be treated, the degree of disease severity, an administration route, a patient weight, an age of the patient, and sex of the patient.

Artificial cartilage or artificial joint having a certain type can be obtained from the cartilage cells that are differentiated according to the method described by using a common method for continuously culturing it on scaffold having a certain type. The artificial joint or the artificial cartilage to be used for a plastic surgery at a specific site of human, such as ears or nose, can be manufactured by using the process described above.

Disclosed is a method for manufacturing artificial joint by using the cartilage cells that are differentiated according to the above method, and the artificial joint manufactured by the above-described method.

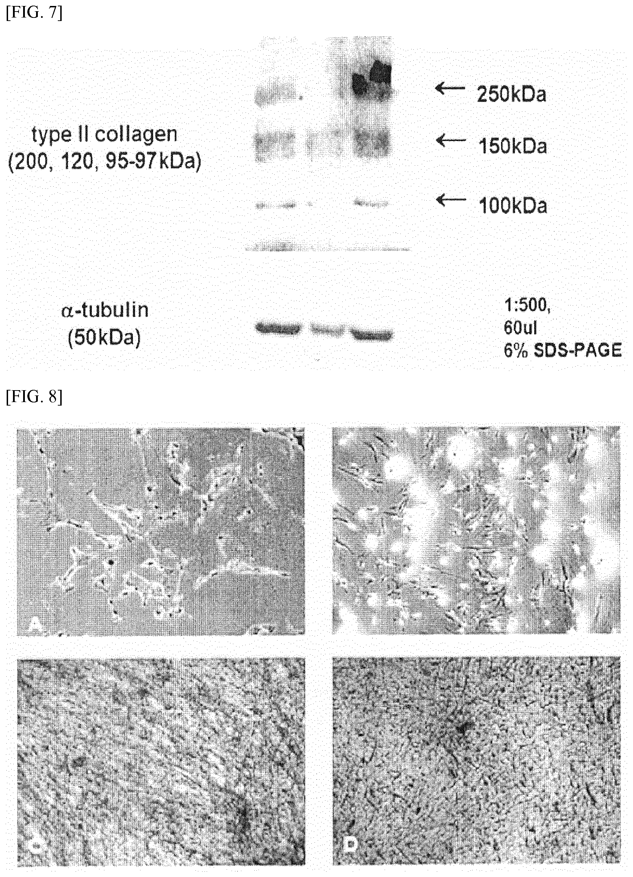

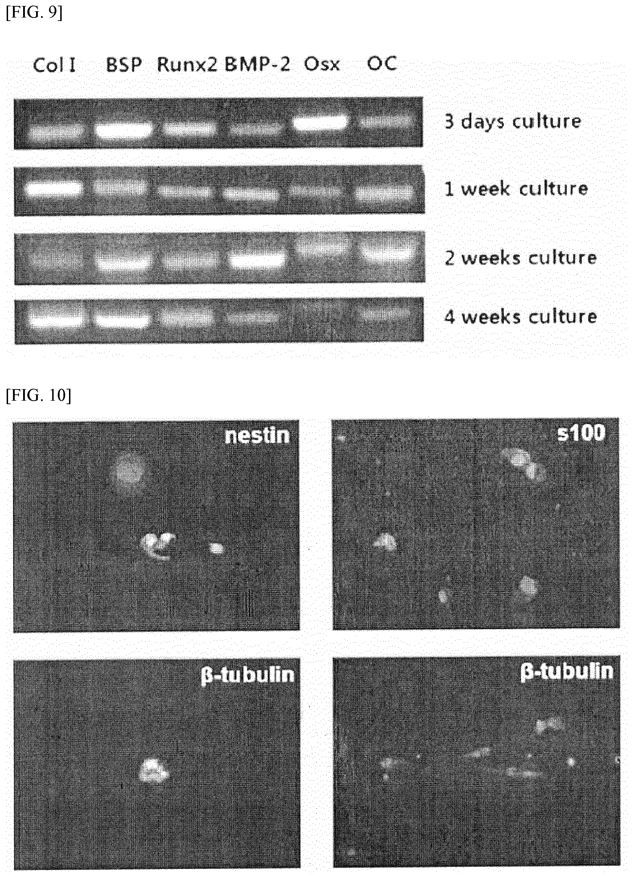

In addition, the inventors investigated the human turbinate mesenchymal stromal cells according to the present invention. Mesenchymal stromal cells that are isolated from human inferior turbinate tissues are cultured in a medium without a fetal bovine serum (FBS) to induce a bone differentiation. As a result, it can be confirmed through performing an alkaline phosphatase staining and an alizarin red staining that the human turbinate mesenchymal stromal cells are differentiated into bone and it can be also confirmed from RT-PCR that a specific gene for a bone differentiation, such as a type I collagen, a bone sialoprotein (BSP), a runt-related transcription factor 2 (Runx2), a bone morphogenetic protein-2 (BMP-2), osterix (Osx), and osteocalcin (OC) are expressed (see FIGS. 8 and 9).

Disclosed is a method for differentiating into bone cells, the method including a step of culturing human turbinate mesenchymal stromal cells (hTMSCs) in a bone differentiation medium.

As the medium for differentiating the human turbinate mesenchymal stromal cells into bone cells, all the mediums are available as long as they are used for culturing animal cells, but is not limited thereto. For example, a DMEM, an alpha-DMEM, an Eagle's basal medium, a RPMI 1640 medium, a Neural progenitor cell basal medium (NPBM, SCIonetics), and the like, may be used, and preferably a DMEM medium without a fetal bovine serum (FBS) may be used.

In addition, the medium used for bone differentiation may further include at least one selected from the group consisting of penicillin, streptomycin, dexamethasone, β-glycerophosphate, and ascorbate-2-phosphate.

Generally, bone forms a frame system using specific joint tissues, supports mechanically body, facilitates muscle activity and movement, and protects internal organs. In addition to the bone function, such as a maintain of body frame and a organ protection, bone controls a biochemical metabolism, and hence bone is dynamic organ because bone causes an active metabolism, thereby continuously repeating steps of formation, resorption, reversal, and formation (Raisv et al. Am J Med, 72; 193-202, 1982). Such the process for repeating the bone resorption and the bone formation refers to a bone remodeling.

In the normal case, osteoclast is activated in human body to resorb and destroy bone, and then osteoblast forms new bone, so that the balance is maintained (Roodman GD, Endocrinol Rev, 17, 308-332, 1996; Boyle W. J. et al., Nature, 423:337-342, 2003). That is, when the bone remodeling occurs by the actions of the osteoblast and osteoclast and then a bone formation amount is equal to a pre-processed bone resorption amount, the dissipation of a bone amount does not occur, and thus a bone amount can be maintained. However, when a bone formation amount is small as compared with a bone resorption amount for some reason or other (when a bone resorption increases or a bone formation amount is smaller than a bone resorption amount), a decrease of bone amount may be caused (Wasnich, R.D. Am J Med, 91; 54s-58s, 1991). In this case, such an imbalance may cause a bone disease.

Accordingly, the bone cells that are differentiated and induced from the human turbinate mesenchymal stromal cells can form bone cells, so that the bone cells can be used as a pharmaceutically composition for treating bone injuries or diseases, or metabolic bone diseases.

The term, "bone injuries or diseases, or metabolic bone diseases" mean diseases causing a physiological pathological condition due to an excessive bone break and resorption. A type of such the diseases may include bone metastatic lesion, in which a tumor, such as osteoporosis, breast cancer, or prostate cancer, spreads to bone; a tumor caused in bone as a primary tumor; a periodontal disease; an inflammatory alveolar bone resorption disease; an inflammatory bone resorption disease; and Paget's disease, but is not limited thereto.

Furthermore, there is disclosed a method for differentiating human turbinate mesenchymal stromal cells into nerve cells, the method including a step of culturing the human turbinate mesenchymal stromal cells in a nerve differentiation medium.

The mesenchymal stromal cells that are isolated from human inferior turbinate tissues are cultured in a medium including nerve growth factors, such as GDNF, BDNF, and NT3, and as a result, it can be found by performing an immune fluorescence staining that the mesenchymal stromal cells are differentiated into nerve cells (see FIG. 10).

As a nerve differentiation medium that is available for differentiating into nerve cells, all the mediums are available as long as they are used for culturing animal cells, and also a medium including nerve growth factors is available.

The nerve growth factors may include a glial-derived neurotrophin factor (GDNF), a brain-derived neurotrophin factor (BDNF), or a neurotrophin-3 (NT3), but is not limited thereto. Above this, a B-27 supplement or L-glutamine may be included.

As mentioned above, based on the fact that human turbinate mesenchymal stromal cells are able to differentiate into nerve cells, a composition for preventing or treating nerve injuries or diseases can be provided, the composition including nerve cells that are differentiated according to the method as an active ingredient.

The nerve injuries or diseases may include a degenerative nerve disease, such as a spinal cord injury, Parkinson's disease, Alzheimer's disease, Huntington's disease, and traumatic central nervous system diseases, but is not limited thereto.

In addition, a composition for preventing or treating nerve injuries or diseases, a composition preventing or treating bone injuries or diseases, or metabolic bone diseases, and a composition for preventing or treating nerve injuries or diseases may separately include cartilage cells, bone cells, or nerve cells that are differentiated and induced from human turbinate mesenchymal stromal cells in a pharmaceutically effective amount, or in combination with at least one of a pharmaceutically acceptable carrier, an excipient, or a diluent. The term "pharmaceutically effective amount" means a sufficient amount for preventing, improving, and treating the above-described diseases.

Pharmaceutically effective amounts of cartilage cells, bone cells, or nerve cells that are differentiated from human turbinate mesenchymal stromal cells, which are included in the above-described compositions, are 0.5 to 100 mg/day/body weight (kg), and preferably 0.5 to 5 mg/day/body weight (kg). However, the pharmaceutically effective amount may be properly controlled according to a symptom degree of such a disease, an age, a body weight, a health condition, and sex of a patient, an administration route, a treatment period, and the like.

Furthermore, from the above-described experiments, the inventors confirmed the fact that all of the human turbinate mesenchymal stromal cells are well differentiated into cartilage cells, bone cells, and nerve cells. Generally, multipotency of mesenchymal stromal cells that come under the spotlight in the prior art is admitted through whether all of three differentiations including fat differentiation as well as cartilage differentiation and bone differentiation are histologically observed.

Accordingly, the inventors observed that human turbinate mesenchymal stromal cells are differentiated into fat cells. As a result, it can be confirmed from the experiment that the human turbinate mesenchymal stromal cells are differentiated into fat cells.

Accordingly, a method for differentiating human turbinate mesenchymal stromal cells into fat cells can be provided, the method including a step of culturing the human turbinate mesenchymal stromal cells in a fat cell differentiation medium.

The method for differentiating into fat cells may include a step of culturing the isolated human turbinate mesenchymal stromal cells in a fat cell differentiation medium including insulin, indomethacin, dexamethasone, 3-isobutyl-1-methylxanthine, and a fetal bovine serum, and preferably in a α-MEM medium including 5 to 15 ml of insulin, 150 to 250 of indomethacin, 0.05 to 1.5 of dexamethasone, 0.2 to 0.7 of 3-isobutyl-1-methylxanthine, and 5 to 15% of a fetal bovine serum for 2 to 4 weeks.

Thus, the fat cells that are differentiated by using the method may be used in a composition for transplanting fat cells for regenerating skin tissues.

Recently, autograft transplantation of fat cells is widely used. Especially, the transplantation of fat is being used in surgery on a lip line, a jaw line, a forehead line, nose, and the like including face lifts of aged skin due to age. In addition, the transplantation of fat shows good result as a surgery for correcting a damaged part by transplanting fat to a collapsed skin due to burns or injuries, a lost part due to carcinomectomy, and the like.

Meanwhile, such a transplantation of fat is being performed by using fat cells that is prepared by removing fibers, blood, and the like from a lump of fat, but the effect is unsatisfactory as far (G Sattler et al., 2000, Dermatol. Sur. 26, 1140-1144).

Fat cells can be used that are differentiated from mesenchymal stromal cells isolated from human inferior turbinate tissues, not fat tissues, as cells transplantation, so that a desired effect can be sufficiently achieved by the transplantation.

Furthermore, fat cells that are differentiated may also be used for beauty treatment purposes for increasing elasticity of skin, improving the wrinkles, particularly, the nasolabial folds of a face, and the glabella Wrinkles of a face, and recovering a dented part thorough a plastic surgery, and may be useful for forming breast tissues, and the like.

In addition, the composition as mentioned may further include a pharmaceutically acceptable carrier. The term, "a pharmaceutically acceptable" means a composition that is physiologically acceptable and does not generally causes an allergic reaction, such as a gastroenteric trouble and dizziness, or a similar reaction thereof when administrating human. The pharmaceutically acceptable carrier may include, for example, an oral-administrated carrier, such as lactose, starch, a cellulose derivative, magnesium stearate, and stearic acid; a parenteral administrated carrier, such as water, suitable oil, saline solution, aqueous glucose, and glycol; and the like, and may further include stabilizers and preserved agents. The suitable stabilizers may include antioxidants, such as sodium bisulfite, sodium sulfite, or ascorbic acid. The suitable preserved agents may include benzalkonium chloride, methyl- or propyl-paraben, and chlorobutanol. As other pharmaceutically acceptable carriers, the carriers as disclosed in the following document can be referred (Remington's Pharmaceutical Sciences, 19th ed., Mack Publishing Company, Easton, PA, 1995).

The composition may be formulated in a suitable type according to a known method in the prior art along with a pharmaceutically acceptable carrier. That is, the composition may be prepared in a type of an oral administration or parenteral administration according to a known method in the prior art, and a representative dosage form for the parenteral administration dosage preferably includes suspension or isotonic aqueous solution in a dosage form for injection. The dosage form for injection may be prepared by using a suitable dispersing agent or wetting agent, and a suspending agent according to a known technique in the prior art. For example, the dosage form for injection may be formulated by dissolving each component in saline solution or a buffer solution. In addition, the dosage form for an oral administration may be powder, granules, tablets, pills, capsules, and the like, but is not limited thereto.

The composition formulated by such a method may be administrated in effective dose through various routes including an oral, a percutaneous injection, a subcutaneous injection, an intravenous injection, or a muscle injection. In this case, the term, "effective dose" means an amount showing a preventive effect or treatment effect when administrating to a patient. A dose of the pharmaceutically composition may be properly selected according to an administration route, an administration object, age, sex, body weight, an individual variation, and a disease condition. Preferably, the pharmaceutically composition may have different content of active ingredients according to the degree of a disease, but may be repeatedly administrated several times a day in preferably 1 to 10000 µg/weight (kg)/day, and more preferably 10 to 1000 mg/weight (kg)/day as an effective dose. In addition, the composition for preventing or treating diseases relating to damages of cartilage cells, bone cells, or nerve cells may be administrated along with known compounds having effects for preventing, improving, or treating such the diseases.

### <Example 1>

### Inducement of Cartilage Differentiation of Human Turbinate Mesenchymal Stromal Cells (hTMSCs)

TGF-β1 and IGF-I that are a standard growth factor of cartilage differentiation, in which the standard growth factor is generally used for a cartilage differentiation of hTMSCs, were used. With the properties of human turbinate mesenchymal stromal cells that are quickly proliferated and human septal cartilage cells that are slowly proliferated and mainly produce a great quantity of a cartilage specific extracellular matrix, the experiments were processed at the same time to increase effectiveness of cartilageinification differentiation by co-culturing human turbinate mesenchymal stromal cells with the septal cartilage cells that are isolated from the septal cartilages of the same person and then cultured. A shape of cell and a histological type of produced extracellular matrix were observed according to an inoculation ratio and culture time and then optimum culture conditions for a clinical use of the cells could be found from the following experiment by confirming productions of cartilage specific stromal components, such as Type II collagen, aggrecan, and the like.

### <1-1> Preparation of Medium for Cartilage Differentiation

A cartilage differentiation medium was prepared by adding 10⁻⁷ M of dexamethasone, 50 µg/ml of ascorbate, 50 mg/ml of insulin-transferrin sodium selenite, 100 µg/ml of sodium pyrubate, 40 µg/ml of praline, and 2 mM of L-glutamine to a Dulbecco's Modified Eagle Media (DMEM, Gibco) including 10% of a fetal bovine serum (FBS). When the medium was replaced at intervals of two or three days, TGF-β1 (10 ng/ml, Sigma) and IGF-I (10 ng/ml, Sigma) were added.

### <1-2> Cell Isolation and Culture

Septal cartilage and inferior turbinate tissues were obtained in a process of performing a septal correction surgery and an inferior turbinate exeresis, and achieved with patient consent before the surgeries to use. Directly after the septal cartilage and inferior turbinate tissues were collected, they were washed three times with physiological saline solution including gentamicin (Kukje Pharm, Seong Nam, Korea) to isolate fibroblast and cartilage cells. Since then, the inferior turbinate tissues removed during a surgery for isolating human turbinate mesenchymal stromal cells were refrigerated at 4°C, and then the tissues were washed three times with an antibiotic-antifungal solution (Gibco, Gaithersberg, MD) at room temperature. Since then, the tissues were again washed two times with a neutral PBS solution and then finely cut in a size of 1 mm³ with a small scissor for a surgery. And then, they were placed in a dish of 100 mm for a culture; covered with a sterile slide glass; adhered to a dish for a culture; added with a DMEM medium including 10% FBS; and then cultured in an incubator under a condition of 37°C and 5% CO₂. After culturing for 3 weeks, the slide glass was removed; cells that are floated in a culture solution were washed to throw out; human turbinate mesenchymal stromal cells that are attached to the floor of the dish for a culture were obtained; subcultured up to three generations; and then used. The isolated human turbinate mesenchymal stromal cells are shown in FIG. 1. In addition, as another method, inferior turbinate tissues that were cut in a size of 1 mm³ were added with a DMEM medium including 0.1 % of collagenase (Sigma, St. Louise, MO); maintained in an incubator under a condition of 37°C and 5% CO₂ for 1 day; and then mesenchymal stromal cells were isolated by performing a centrifugation.

In order to isolate human septal cartilage cells, septal cartilage tissue pieces removed during a surgery process were refrigerated; and then washed three times with an antibiotic-antifungal solution at room temperature. Since then, the tissue pieces were again washed two times with a neutral PBS solution and then the septal cartilage was finely cut in a size of 1 mm³. And then, they were placed in a dish for a culture; 20 ml of a DMEM (Dulbecco's Modified Eagle Media, Gibco) including 10% of a fetal bovine serum (FBS) was added to the dish for a culture; 0.3% of collagenase (Sigma, St. Louise, MO) was added; and then cultured in a cell incubator of 37°C for 12 hours. Since then, it was centrifuged at 1000 rpm for 10 minutes; the supernatant was removed; and then washed with PBS two times to obtain cartilage cells. The cartilage cells was inoculated in T75 flask (NUNC, Roskilde, Denmark), and cultured in a cell incubator of 37°C and 5% CO₂. In this case, the culture solution was changed per two to three days.

### <1-3> Cell Inoculation and Three-Dimensional Culture

A three-dimensional culture was performed by using a PCL (polycaprolactone) sponge that is a standardized three-dimensional scaffold as a scaffold, in which the PCL sponge was manufactured using micro-stereolithography to a 24-well plate (NUNC Company). That is, the cartilage cells and fibroblast isolated in the above-described example were calculated in 2 x 10⁶/ml of a cell number; and human turbinate mesenchymal stromal cells and septal cartilage cells of the same person were inoculated in a ratio of 1:0, 1:1, and 0:1, respectively. A DMEM medium including TGF-β1(10 ng/ml, Sigma) and IGF-I (10 ng/ml, Sigma) as a growth factor and 10% FBS was added. The medium was replaced at intervals of three days for a period for culturing; a culture scaffold was taken at 2 weeks for culturing; and then the differentiation into cartilage cells was observed through a histological method, an immunohistochemical method, a western blot, and a RT-PCR method.

### <1-4> Analysis using Histologic Method

The culture scaffold after 2 weeks of culturing in the method of <1-3> was fixed with 10% formalin according to a common histological method; embedded in paraffin; a section having 5 µm of a thickness was prepared; and then the prepared specimen was histologically observed with a microscope after performing a H & E staining and a toluidine blue-O staining. And then, a distal image was taken using Spot software (Polaroid Digital Microscope Camera le v1.25, Polaroid, Waltham, MA).

### <1-5> Analysis using Immunohistochemical method to Type II Collagen

A section of the same culture scaffold as the scaffold used in the histological method was attached to an object glass and then hydrated; added to a TBS solution including 1.45 units/ml chondroitinase (Sigma) and 0.5 units/ml testicular hyaluronidase (Sigma); treated at 37°C for 1 hour; and then treated with a TBS including 0.3% peroxide for 30 minutes so as to remove activity of endogenous peroxidase. The culture scaffold section that was treated with the process described above was reacted in a 10% horse serum for 20 minutes and then the serum was removed; and then a primary antibody (Sigma) to human Type II collagen was reacted for 8 hours at room temperature. After this, all of the primary antibodies to be double-stained were reacted overnight; and then washed three times with a PBS including a 3% BSA. Alexa 555 that is a secondary antibody was treated in a ratio of 1:100 for 1 hour at room temperature and then confirmed whether or not the expression was shown. In order for a nucleus staining, it was fixed with a DAPI-containing mounting medium (Vectashild, Burlingame, CA). The cells were confirmed to check whether or not the expression was shown by using a fluorescence microscope (Olympus AX70TR62A02, Tokyo, Japan).

As a result, it could be found based on the histological opinion of the culture scaffold collected at 2 weeks of a cartilage differentiation culture of the human turbinate mesenchymal stromal cells that the human turbinate mesenchymal stromal cells that were stimulated with a growth factor, such as TGF-β1 and IGF-I showed a shape of tissue mass, in which lesser tubercles that was formed by collecting the cells in spaces of the PCL sponge used as a scaffold was expanded to sparsely arrange the cells between stromas (see FIG. 2). In addition, it could be observed as the red stained parts by performing a toluidine blue staining and could be confirmed as the red stained parts in the immunehistological staining to Type II collagen that human turbinate mesenchymal stromal cells generated cartilage specific matrices.

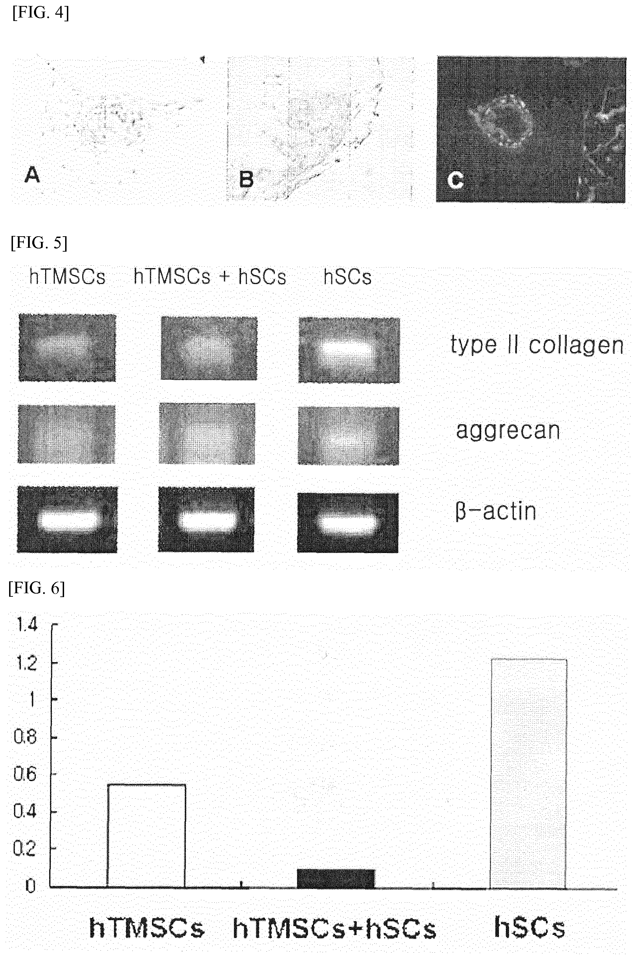

In addition, the result about tissues generating the cartilage specific matrices described above was also observed in an experiment group that was co-cultured with septal cartilage cells of the same person. The above result was almost the same as the group that was cultured only with septal cartilage cells only used for a positive control group (see FIGS. 3 and 4).

### <1-6> RNA Isolation and Reverse Transcriptase Polymerase Chain Reaction

The same culture scaffold as the scaffold used for the histological method was washed with PBS and RNA was extracted according to the method that was suggested by a manufacturer by adding 1 ml of a Tri-Zol™ reagent (Invitrogen, Carlsbad, CA) using Ambion RNAqueous kit (Ambion, Austin, TX). That is, the culture scaffold was ground using a homogenizer (Polytron, Luzern, Switzerland); put in a sterilized container; and then mixed by using a Rocker (VS-95RK, Vision, Korea) for 10 minutes at room temperature. 0.2 ml of chloroform (Sigma) was added to the mixed culture scaffold to mix; maintained at room temperature for 3 minutes; the supernatant was isolated by centrifuging at 14000 rpm for 15 minutes; 0.5 ml of isopropanol (Sigma) was added to the isolated supernatant; maintained at room temperature for 10 minutes; the pellet was obtained by centrifuging at 14000 rpm for 15 minutes; and then the pellet was washed with 70% ethanol. 30 µl of water without RNase was added to the washed pellet and then maintained in an oven of 55°C to 65°C for 10 minutes to isolate RNA.
An oligo dT-primed cDNA synthesis was performed by using MMLV-Reverse Transcriptase (MMLVRT, Invitrogen) and PCR amplification of cDNA was performed by using type II collagen and aggrecan primer. The base sequences of the used primers are shown in the following Table 1.

**[Table 1]**

| Primer Sequence | | | |
|---|---|---|---|
| Genes | Primer Sequence | PCR Product Size (base pairs) | SEQ. ID. NO. |
| β-actin (Forward) | 5'-GCCCCTCCATCGTCCACCGC-3' | 493 | 1 |
| β-actin (Reverse) | 5'-GGGCACGAAGGCTCATCATT-3' | 493 | 2 |
| type II collagen (Forward) | 5'-TTTCCCAGGTCAAGATGGTC-3' | 377 | 3 |
| type II collagen (Reverse) | 5'-CTTCAGCACCTGTCTCACCA-3' | 377 | 4 |
| Aggrecan (Forward) | 5'-GCAGAGACGCATCTAGAAATT-3' | 507 | 5 |
| Aggrecan (Reverse) | 5'-GGTAATTGCAGGGAACATCATT-3' | 507 | 6 |

As a result, as illustrated in FIG. 5, it could be found by performing RT-PCR method that mRNA to β-actin used as a positive control group and mRNAs to type II collagen and aggrecan were expressed at 2 weeks of culture. As a result of a quantitative analysis by using RT-PCR, it could be confirmed that the degree of cartilage differentiation of human turbinate mesenchymal stromal cells was similar to that when culturing only septal cartilage cells. Accordingly, it could be found that the human turbinate mesenchymal stromal cells according to the present invention can be differentiated into cartilage cells.

### <1-7> Analysis using Western Blot

The same culture scaffold as the scaffold used for the histological method was homogenized by putting in 1 ml of a RIPA (RadiolmmunoPrecipitation) buffer (150 mM of NaCl, 1% of NP-40, 50 mM of Tris(pH 8.0), 1 mM of EDTA, 0.5% of Deoxycholate) including a protein degradation inhibitor (100 µg/ml of phenylmethylsulfonyl fluoride, 1 µg/ml of leupeptin). The homogenate was centrifuged at 15,000 rpm for 10 minutes to obtain supernatant; the supernatant was used for a western blot analysis; the amount of protein was measured by using a Bio-Rad protein analysis (Bio-Rad, Hercules, CA, USA). The quantitatively measured proteins were electrophoresed by using SDS-PAGE, transferred to a nitrocellulose filter (Amersham,Arlington Heights, IL, USA), and then reacted with anti-alkaline phostatase and osteocalcin antiboides at 4°C for 12 hours. Since then, the filter was washed with a TBS solution including 0.1% of Tween-20, reacted with peroxidase-conjugated donkey anti-rabbit antibody (Amersham) and donkey anti-mouse antibody (Amersham), respectively, and then confirmed with X-ray film by using a ECL solution (Amersham).

As a result, as illustrated in FIG. 7, it could be found that Type II collagen was actually produced from the human turbinate mesenchymal stromal cells that are isolated in the present invention.

### <Example 2>

### Inducement of Bone Differentiation from Human Turbinate Mesenchymal Stromal Cells (hTMSCs)

By confirming that cartilage could be differentiated by using human turbinate mesenchymal stromal cells through Example 1 described above, the inventors performed the experiment about an inducement of bone differentiation by using human turbinate mesenchymal stromal cells as follows.

### <2-1> Preparation of Bone Differentiation Medium

As a bone differentiation medium, 100 U/ml of penicillin, 100 µg/ml of streptomycin, 10⁻⁸ M of dexamethasone, 5 mM of β-glycerophosphate, and 50 µg/ml of ascorbate-2-phosphate were added to a DMEM medium without FBS. Such a medium was used as the bone differentiation medium.

### <2-2> Cell Inoculation and Culture

The number of human turbinate mesenchymal stromal cells were counted in 2 x 10⁴/ml, and then were inoculated into a 12-well plate. Since then, the difference of the degree of the bone differentiation was confirmed by adding 10 nM and 100 nM concentrations of 1,25-dihydroxyvitamin D₃ and 100 ng/ml of BMP-2 (bone morphogenic protein) to the medium while replacing with the bone differentiation medium prepared in the <1-1> at intervals of two days for a culturing period.

### <2-3> Alizarin Red S Staining

Each of the wells including the cells that are cultured in the <2-2> was washed one time with PBS, washed three times with sterilized triple distilled water, and then fixed with 4% of paraformaldehyde for 15 minutes. Since then, each of the walls was washed three times with sterilized triple distilled water, and then cultured and stained with 2% of Alizarin Red S solution (1 ml of 40 mM Alizarin Red S solution) for 20 minutes at room temperature while gently shaking in the state of blocking light. Unincorporated dye was aspirated, washed four times with 4 ml of sterilized triple distilled water per a well for 5 minutes while shaking, and then observed with an inverted microscope.

### <2-4> Akaline Phosphatase Staining

Each of the wells including the cells that are cultured in the <2-2> washed one time with PBS, washed three times with sterilized triple distilled water, and then fixed with a citrate-acetone-formaldehyde fixing solution for 15 minutes. Each of the wells washed three times with sterilized triple distilled water, stained with alkaline dye mixing solution (Nitrite, FRV-alkaline, Naphthol AS-BI alkaline siolution, Sigma) for 15 minutes at room temperature in the state of blocking light, washed three times with sterilized triple distilled water, and the observed under an inverted microscope.

As the results of performing an Alizarin Red S staining and an alkaline phosphatase staining, as illustrated in FIG. 8, it was found that bone cells were differentiated from the human turbinate mesenchymal stromal cells.

### <2-5> RNA Isolation and Reverse Transcriptase Polymerase Chain Reaction

The cells that are cultured for a bone differentiation was washed with PBS, and extracted by adding 1 ml of a Tri-Zol™ reagent using an Ambion RNAqueous kit according to a method suggested by a manufacturer. In order to confirm bone differentiation-specific mRNA, type I collagen, bone sialoprotein (BSP), Runt-related transcription factor 2 (Runx2), bone morphogenetic protein-2 (BMP-2), osterix (Osx), and osteocalcin (OC) primers were manufactured and a common RT-PCR method in the prior art is used. The primers sequences used are shown in the following Table 2.

**[Table 2]**

| Primer Sequence | | | |
|---|---|---|---|
| Gene | Primer | PCR Product (base pairs) | SEQ. ID. NO. |
| COL 1 (Forward) | 5'-TGACGAGACCAAGAACTG-3' | 599 | 7 |
| COL 1 (Reverse) | 5`-CCATCCAAACCACTGAAACC-3' | 599 | 8 |
| OC (Forward) | 5'-ATGAGAGCCCTCACACTCCTC-3' | 297 | 9 |
| OC (Reverse) | 5'-CGGGCCGTAGAAGCGCCGATA-3' | 297 | 10 |
| BSP (Forward) | 5'-GCTCAGCATTTTGGGAATGGC-3' | 614 | 11 |
| BSP (Reverse) | 5'-CTGCATTGGCTCCAGTGACAC-3' | 614 | 12 |
| Runx2 (Forward) | 5'-GTGGACGAGGCAAGAGTTTCA-3' | 698 | 13 |
| Runx2 (Reverse) | 5'-TGGCAGGTAGGTGTGGTAGTG-3' | 698 | 14 |
| Osx (Forward) | 5'-CTTCAGTCTTCCCAACTTCTTACAC-3' | 486 | 15 |
| Osx (Reverse) | 5'-ACAAATTGGGTTAGCTACATCTCTG-3' | 486 | 16 |
| BMP-2 (Forward) | 5'-TTGCGCCTGCTCAGCATGTT-3' | 315 | 17 |
| BMP-2 (Reverse) | 5'-CATCTTGCATCTGTTCTCGGAA-3' | 315 | 18 |

As a result, as illustrated in FIG. 9, RT-PCR was performed by using the differentiated cells as a target, and thus bone differentiation-specific genes were expressed.

Accordingly, it was found from the results described above that the human turbinate mesenchymal stromal cells according to the present invention could induce a bone differentiation.

### <Example 3>

### Inducement of Nerve Differentiation from Human Turbinate Mesenchymal Stromal Cells (hTMSCs)

### <3-1> Inducement of Differentiation into Nerve Cells

Furthermore, in order to investigate whether or not human turbinate mesenchymal stromal cells are available for differentiating into nerve cells, a neurobasal medium was used as a basic medium, the cells were cultured for 14 days using 10 ng/ml of a Glial-derived neurotrophin factor (GDNF) and 10 ng/ml of a Brain-derived neurotrophin factor (BDNF) as a growth factor, 10 ng/ml of an Neurotrophin-3 (NT3), 1 ml of B-27 supplement, and 2 mM L-glutamin were used while replacing the medium at intervals of three days.

### <3-2> Analysis using Immunocytochemistry

Human turbinate mesenchymal stromal cells were subcultured three times to use for an experiment. The cells were counted in 1 × 10⁴ cells/well, inoculated on a Lab-Tec 4-well cahmber slide (Nalgene Nunc International, Rochester, NY), and then induced for a nerve differentiation with the medium of the <3-1>. The cells were labeled with BrdU (5-Bromo-2'-deoxy-uridine Labeling and Detedtion Kit, Roche, Indianapolis, IN) as a marker of amplified cell for 24 hours. The labeled cells were washed three times with PBS including 3% BSA (Bovine serum qlbumin, 12 Williams Ave Keilor East, VIC 3033, Australia), fixed with 4% paraformaldehyde for 20 minutes, and then treated with 0.5% Triton X-100 (Promega Co. Medison, WI) for 20 minutes at room temperature. Since then, an anti-BrdU working solution (1:10) that is a primary antibody were treated for equal to or greater than 12 hours, washed three time with PBS including 3% BSA, and then an anti-mouse-Ig-Fluorescein working solution (1:10) that is a secondary antibody were treated at room temperature for 1 hour. Since then, after blocking with 5% normal goat solution, a double staining was performing by using S-100 (Abcam) that is glial cells marker, b-tubulin (Abcam) that is nerve cells marker, and Nestin (Abcam) that is a neural stem cell marker, as a specific marker for a respective typically differentiated cell. All of the primary antibodies to be double-stained were reacted overnight, and then washed three times with PBS including 3% BSA. Alexa 555 that is a secondary antibody was treated in a ratio of 1:100 at room temperature for 1 hour, and then confirmed whether or not the expression was shown. In order to stain nucleus, the cells were fixed to a DAPI-conjugated mounting medium (Vectashild, Burlingame, CA), and the expression was confirmed by using a fluorescence microscope (Olympus AX70TR62A02, Tokyo, Japan).

As a result, as illustrated in FIG. 10, it could be confirmed from the result of culturing the human turbinate mesenchymal stromal cells used in the present invention on the culturing medium that the cells were differentiated into nerve cells through an observation of the nerve cell markers.

### <Example 4>

### Confirmation of Properties of Mesenchymal Stromal Cells using Fluorescence Active Cell analysis

In order to confirm that human turbinate mesenchymal stromal cells have properties of mesenchymal stem cells, it was analyzed by using human stem cell surface marker. In order for the analysis, three times subcultured human turbinate mesenchymal stromal cells were counted in 1 × 10⁶ cells/ml; the cells were seeded to a test tube, and then washed three times with a washing buffer solution (0.2% of BSA, 0.1% of NAN₃, 0.5mM EDTA). Since then, the cells were treated with fluorescein isothicyanate (FITC) and phycoerythrin (PE)-conjugated CD34 (Serotec Ltd., Station Approach, Kidlington, Oxford, UK), CD45 (Serotec Ltd.), CD73, CD90, and CD105 (all from BD Bioscience, San Jose, CA) antibodies for 1 hour, washed three times with a washing buffer solution, and then analyzed using a flow cytometer (FACS Caliber, Becton Dickson, San Diego, CA) apparatus.

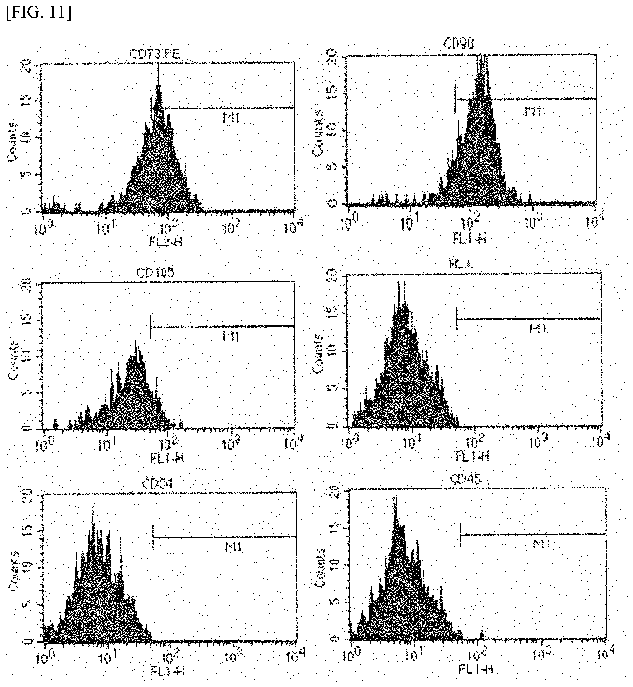

As a result, as illustrated in FIG. 11, a surface marker, such as CD34 and CD45 that are hematopoietic stem cell markers, was confirmed in almost 100% negative and surface markers, that is, CD73, CD90, and CD105 that is known as a surface marker of mesenchymal stromal cells were 65.45%, 91.44%, and 7.78% as a positive reaction, respectively. Accordingly, it could be found from the result described above that the human turbinate mesenchymal stromal cells used in the present invention had properties of mesenchymal stromal cells. In addition, it could be found by confirming 100% negative to HLA (Human leukocyte antigen) surface markers serving as histocompatibility antigens that there were no immunological problems about the suitability of human body.

### <Example 5>

### Inducement of Differentiation of Human Turbinate Mesenchymal Stromal Cells (hTMSCs) into Fat Cells

The inventors could confirm multipontency of the stromal cells by well performing cartilage and bone differentiations of human turbinate mesenchymal stromal cells through the experiments. Meanwhile, it comes under the spotlight in the prior art that multipotency of mesenchymal stromal cells is admitted through whether all of three differentiations including fat differentiation as well as cartilage differentiation and bone differentiation are histologically observed.

Accordingly, the inventors performed the following experiment in order to differentiate human turbinate mesenchymal stromal cells into fat cells.

The human turbinate mesenchymal stromal cells obtained from Example described above were seeded to a 6-well plate to be the number of 3 × 10³ cells and then cultured in a α-MEM (GIBCO) medium including 10% of a fetal bovine serum and amtobiotics for 3 days. Since then, the cells were incubated in a fat cell differentiation medium (a α-MEM medium including 10/ml of insulin (Sigma), 200 of indomethacin (Sigma), 0.1 of dexamethasone (Sigma), 0.5 of 3-isobutyl-1-methylxanthine (Sigma), and 10% fetal bovine serum) for 3 weeks, and an oil red staining was performed to observe whether or not the human turbinate mesenchymal stromal cells were differentiated into fat cells. In addition, the oil red staining was performed by fixing the cultured cells with 4% of paraformaldehyde for 30 minutes, washing, staining with 0.16% of an oil red O reagent for 20 minutes, and then observing through a microscope.

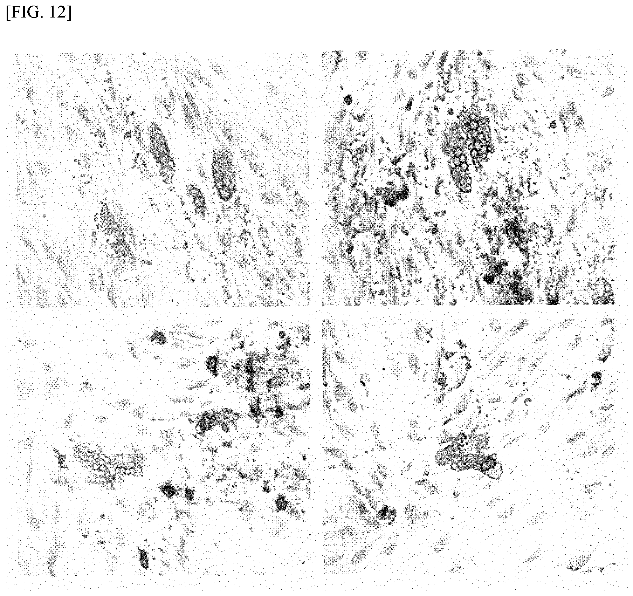

As a result, as illustrated in FIG. 12, it could be found that the human turbinate mesenchymal stromal cells isolated in the present invention were differentiated into fat cells and in the case of the differentiated cells; there were lipid droplets stained with orange color in a fat specific cytoplasm.
## Claims

1. A method for differentiating human turbinate mesenchymal stromal cells (hTMSCs) into bone, nerve or fat cells, the method comprising a step of culturing isolated human inferior turbinate tissues; a step of culturing the inferior turbinate tissues by adhering the inferior turbinate tissues to a culture dish; a step of removing un-adhered cells; a step of obtaining human turbinate mesenchymal stromal cells adhered on the dish; a step of sub-culturing 3 times; and a step of sub-culturing human turbinate mesenchymal stromal cells in a bone, nerve or fat cell differentiation medium.

2. The method according to claim 1, wherein the human turbinate mesenchymal stromal cells are differentiated into bone cells by culturing the human turbinate mesenchymal stromal cells in a medium without a fetal bovine serum (FBS).

3. The method according to claim 2, wherein the medium further includes at least one selected from the group consisting of penicillin, streptomycin, dexamethasone, β-glycerophosphate, and ascorbate-2-phosphate.

4. The method according to claim 1, wherein the human turbinate mesenchymal stromal cells are differentiated into nerve cells by culturing the human turbinate mesenchymal stromal cells in a nerve differentiation medium including a glial-derived neurotrophin factor (GDNF), a brain-derived neurotrophin factor (BDNF), a neurotrophin-3 (NT3), a B-27 supplement, and L- glutamine.

5. The method according to claim 1, wherein the human turbinate mesenchymal stromal cells are differentiated into fat cells by culturing the human turbinate mesenchymal stromal cells in a fat cell differentiation medium including insulin, indomethacin, dexamethasone, 3-isobutyl-1-methylxanthine, and a fetal bovine serum (FBS).

## Patentansprüche

1. Verfahren zum Differenzieren von humanen mesenchymalen Stromazellen aus der Nasenmuschel (human Turbinate Mesenchymal Stromal Cells, hTMSCs) zu Knochen-, Nerven- oder Fettzellen, wobei das Verfahren einen Schritt, bei dem man isolierte humane Gewebe der unteren Nasenmuschel kultiviert, einen Schritt, bei dem man Gewebe der unteren Nasenmuschel kultiviert, indem man die Gewebe der unteren Nasenmuschel an eine Kulturschale anheftet, einen Schritt, bei dem man nicht angeheftete Zellen entfernt, einen Schritt, bei dem man humane mesenchymale Stromazellen aus der Nasenmuschel, die an der Schale angeheftet sind, gewinnt, einen Schritt, bei dem man dreimal subkultiviert, und einen Schritt, bei dem man humane mesenchymale Stromazellen aus der Nasenmuschel in ein Knochen-, Nerven- oder Fettzelldifferenzierungsmedium subkultiviert, umfasst.

2. Verfahren nach Anspruch 1, wobei die humanen mesenchymalen Stromazellen aus der Nasenmuschel zu Knochenzellen differenziert werden, indem man die humanen mesenchymalen Stromazellen aus der Nasenmuschel in einem Medium ohne fetales Rinderserum (Fetal Bovine Serum, FBS) kultiviert.

3. Verfahren nach Anspruch 2, wobei das Medium weiterhin mindestens eine Verbindung ausgewählt aus der Gruppe bestehend aus Penicillin, Streptomycin, Dexamethason, β-Glycerophosphat und Ascorbat-2-phosphat umfasst.

4. Verfahren nach Anspruch 1, wobei die humanen mesenchymalen Stromazellen aus der Nasenmuschel zu Nervenzellen differenziert werden, indem man die humanen mesenchymalen Stromazellen aus der Nasenmuschel in einem Nervendifferenzierungsmedium kultiviert, welches einen Glial-Derived Neurotrophin Factor (GDNF), einen Brain-Derived Neurotrophin Factor (BDNF), ein Neurotrophin-3 (NT3), einen B-27-Zusatz und L-Glutamin umfasst.

5. Verfahren nach Anspruch 1, bei dem man die humanen mesenchymalen Stromazellen aus der Nasenmuschel zu Fettzellen differenziert, indem man die humanen mesenchymalen Stromazellen aus der Nasenmuschel in einem Fettzelldifferenzierungsmedium kultiviert, welches Insulin, Indomethacin, Dexamethason, 3-Isobutyl-1-methylxanthin und fetales Rinderserum (FBS) umfasst.

## Revendications

1. Méthode de différenciation de cellules stromales mésenchymateuses humaines du cornet (hTMSC) en cellules osseuses, nerveuses ou adipeuses, la méthode comprenant une étape de culture de tissus humains isolés du cornet inférieur ; une étape de culture des tissus du cornet inférieur en faisant adhérer les tissus du cornet inférieur à une boîte de culture ; une étape d'élimination des cellules n'ayant pas adhéré ; une étape d'obtention de cellules stromales mésenchymateuses humaines du cornet ayant adhéré à la boîte ; une étape de mise en sous-culture 3 fois ; et une étape de mise en sous-culture de cellules stromales mésenchymateuses humaines du cornet dans un milieu de différenciation en cellules osseuses, nerveuses ou adipeuses.

2. Méthode selon la revendication 1, où les cellules stromales mésenchymateuses humaines du cornet sont différenciées en cellules osseuses par mise en culture des cellules stromales mésenchymateuses humaines du cornet dans un milieu sans sérum foetal bovin (FBS).

3. Méthode selon la revendication 2, où le milieu inclut en outre au moins l'un de ceux qui sont choisis dans le groupe constitué par pénicilline, streptomycine, dexaméthasone, β-glycérophosphate et ascorbate-2-phosphate.

4. Méthode selon la revendication 1, où les cellules stromales mésenchymateuses humaines du cornet sont différenciées en cellules nerveuses par mise en culture des cellules stromales mésenchymateuses humaines du cornet dans un milieu de différenciation en cellules nerveuses incluant un facteur neurotrophique dérivé des cellules gliales (GDNF), un facteur neurotrophique dérivé du cerveau (BDNF), une neurotrophine-3 (NT3), un supplément B-27, et la L-glutamine.

5. Méthode selon la revendication 1, où les cellules stromales mésenchymateuses humaines du cornet sont différenciées en cellules adipeuses par mise en culture des cellules stromales mésenchymateuses humaines du cornet dans un milieu de différenciation en cellules adipeuses incluant insuline, indométhacine, dexaméthasone, 3-isobutyl-1-méthylxanthine et sérum foetal bovin (FBS).
